# EUROPEAN PATENT APPLICATION

(11) **EP 3 150 242 A1**
(43) Date of publication of application: **05.04.2017**
(21) Application number: 15187805.5
(22) Date of filing: 30.09.2015
(51) Int. Cl.: A61M 5/44, A61M 5/168, A61F 7/00

(54) **TEMPERATURE ADJUSTMENT INFUSION SYSTEM AND METHOD**

(71) Applicant: seiratherm GmbH, 91074 Herzogenaurach (DE)
(72) Inventor: Roth, Matthias, 85376 Giggenhausen (DE); Reichthalhammer, Thomas, 85716 Unterschleissheim (DE)
(74) Representative: Stellbrink & Partner Patentanwälte mbB

(57) **Abstract**

The present invention is directed to a device and method, particularly suitable in a temperature adjustment infusion system and more particularly suitable for normothermia and/or hypothermia. The device comprises at least one connection (11) to a reservoir (10) suitable to provide infusion fluid; at least one temperature controller (30) adapted to cool and/or heat the temperature of the infusion fluid so that the infusion fluid is delivered with a pre-set temperature at a temperature of between -1°C and 42°C; at least one flow controller (40) adapted to control the flow downstream the temperature controller (30) in an average amount of between 100 ml and 8000 ml with an average flow rate of preferably 40 ml/h to 8000 ml/h; and at least one output (14) adapted to deliver the infusion fluid downstream the reservoir.

## Description

### Field

The invention concerns a temperature adaption or adjustment infusion system, particularly a normothermia system and/or a hypothermia system, and respective methods.

### Introduction

Normal human body temperature, also known as normothermia or euthermia, depends upon the place in the body at which the measurement is made, the time of day, as well as the activity level of the person. Nevertheless, commonly mentioned typical values are oral (under the tongue): 36.8±0.4 °C (98.2±0.72 °F) or internal (rectal, vaginal): 37.0 °C (98.6 °F). Different parts of the body have different temperatures. Rectal and vaginal measurements taken directly inside the body cavity are typically slightly higher than oral measurements, and oral measurements are somewhat higher than skin measurements. Other places, such as under the arm or in the ear, produce different typical temperatures.

The body temperature of a healthy person varies during the day by about 0.5 °C (0.9 °F) with lower temperatures in the morning and higher temperatures in the late afternoon and evening, as the body's needs and activities change. Other circumstances also affect the body's temperature. The core body temperature of an individual tends to have the lowest value in the second half of the sleep cycle; the lowest point, called the nadir, is one of the primary markers for circadian rhythms. The body temperature also changes when a person is hungry, sleepy, sick, or cold.

Temperature control (thermoregulation) is part of a homeostatic mechanism that keeps the organism at optimum operating temperature, as it affects the rate of chemical reactions.

Fever of a human being, also known as pyrexia and febrile response, is defined as having a temperature above the normal range due to an increase in the body's temperature set-point. Upper limits for normal temperature can (but must not) be values between 37.5 and 38.3 °C (99.5 and 100.9 °F).The increase in set point triggers increased muscle contraction and causes a feeling of cold. This results in greater heat production and efforts to conserve heat. When the set-point temperature returns to normal a person feels hot, becomes flushed, and may begin to sweat. Rarely a fever may trigger a febrile seizure. This is more common in young children. Fevers do not typically go higher than 41 to 42 °C (105.8 to 107.6 °F).

A fever can be caused by many medical conditions ranging from the not serious to potentially serious. This includes viral, bacterial and parasitic infections such as the common cold, urinary tract infections, meningitis, malaria and appendicitis among others. Non-infectious causes include vasculitis, deep vein thrombosis, side effects of medication, and cancer among others. It differs from hyperthermia, in that hyperthermia is an increase in body temperature over the temperature set-point, due to either too much heat production or not enough heat loss.

Treatment to reduce fever, particularly high fever, is required in many cases. Treatment, may increase comfort and help a person rest or may even be a life or health saving requirement. Hyperthermia may also require treatment.

Hypothermia is usually called a condition in which the body's core temperature drops below that required for normal metabolism and body functions. This is generally considered to be less than 35.0 °C (95.0 °F). Characteristic symptoms depend on the temperature. Targeted temperature management (TTM) previously known as therapeutic hypothermia or protective hypothermia is active treatment that tries to achieve and maintain a specific body temperature in a person for a specific duration of time in an effort to improve health outcomes. This is done in an attempt to reduce the risk of tissue injury from lack of blood flow. Periods of poor blood flow may be due to cardiac arrest or the blockage of an artery by a clot such as may occur in stroke. Targeted temperature management improves survival and brain function following resuscitation from cardiac arrest. Evidence supports its use following ROSC (return of spontaneous circulation) after cardiac arrest. Targeted temperature management following traumatic brain injury has shown mixed results with some studies showing benefits in survival and brain function while other show no clear benefit. While associated with some complications, these are generally mild. Targeted temperature management can advantageously prevent brain injury by several methods including decreasing the brain's oxygen demand, reducing the production of neurotransmitters like glutamate, as well as reducing free radicals that might damage the brain. The lowering of body temperature may be accomplished by many means including the use of cooling blankets, cooling helmets, cooling catheters, ice packs and ice water lavage.

Medical events that targeted temperature management may effectively treat fall into five primary categories: neonatal encephalopathy, cardiac arrest, ischemic stroke, traumatic brain or spinal cord injury without fever, and any fever, e.g., neurogenic fever following brain trauma.

US 2004 059400 A discloses a fever relief device with a body in which a thermoelectric cooler is received and the assembly of the body and the cooler is conveniently mounted to the head of the user who may adjust the direct current to control the temperature of the cooler so as to relieve the fever.

US 4,845,788 A is directed to a water fillable mattress, with a support, has water circulating passages and an inflatable cover, releasably attachable to one side of the mattress, and permanently attached to another side of the mattress. The mattress is sized to support a child and is adapted to relieve the fever of the child when cold water is circulated through its passages.

US 8,480,648 B1 discloses an automated therapy system having an infusion catheter, a sensor adapted to sense a patient parameter, and at least one assembly controller communicating with the sensor and programmed to control flow output from the infusion catheter into a patient based on the patient parameter without removing fluid from the patient. This US document also includes a method of controlling infusion of a fluid to a patient. The method includes the following steps: monitoring a patient parameter with a sensor to generate a sensor signal; providing the sensor signal to at least one assembly controller; and adjusting fluid flow to the patient based on the sensor signal without removing fluid from the patient.

EP 2514453 B1 relates to a device and method for controlling a temperature of a patient by an infusion of fluid. Said device comprises a supply of infusion fluid, a body temperature input adapted to receive the actual body temperature of the patient and an additional input adapted to receive at least one additional parameter representing the actual physiological state of the patient. Furthermore, the device comprises a control unit communicating with said body temperature input, and said additional input and at least one actuator which is in fluid communication with said supply and which controls the actual flow rate and/or actual temperature of the infusion fluid in accordance with at least one control signal of said control unit.

US 7,867,188 B2 shows a disposable warmer cartridge that is used to heat fluids to be infused to the patient to prevent hypothermia in the patient. The cartridge has in its chamber a pair of spaced in parallel electrodes that have substantially the same dimension. When RF power is fed to the electrodes, an alternating electric field is generated between the electrodes to directly heat the fluid that is in the chamber. The heating of the fluid is achieved in a substantially instantaneous manner by controlling the energization of the electrodes through the distributed impedance of the electric field between the electrodes. Modulating the RF power fed to the electrodes readily controls heat. Feedback to control the temperature of the fluid in the cartridge may be provided by non-contact and direct contact sensors.

EP 2698182 A1 relates to a method and a device for adjusting the temperature of medical liquids, comprising providing an incoming volumetric flow from a fluid supply, separating the incoming volumetric flow into two partial volumetric flows. Further, the fluid temperature of each of the partial volumetric flows is adjusted to substantially constant target temperatures of the partial volumetric flows; and volumetric flow controlled merging of the partial volumetric flows to an output volumetric flow.

### Summary of the Invention

The problem underlying the present invention is to provide an alternative or ameliorated normothermia and/or hyperthermia device or system and method for infusion fluids.

The problem is solved by the subject matter of the present invention exemplified by the description and the claims.

The present invention is directed to a device and method, particularly suitable for normothermia and/or hypothermia. The device can comprise at least one connection to a reservoir suitable to provide infusion fluid. At least one temperature controller can be adapted to cool the temperature of the infusion fluid sufficiently so that the infusion fluid is delivered with a pre-set or given temperature at a temperature of between -1°C and 40°C, At least one flow controller can be adapted to control the flow downstream the temperature controller in an average amount of between 100 ml/h and 10 l/h. Further at least one output is provided and adapted to deliver the infusion fluid downstream the reservoir.

The infusion fluid can be any among known fluids such as blood/blood derivates, pharmacological fluids, nutritional fluids, and fluid infusion systems and/or an infusion system for infusing, e.g., saline or other balanced fluids like ringer's solution. Also the kind, shape, material and volume can vary.

Further at least one temperature sensor can be suitable to deliver at least one temperature signal at least one assembly controller adapted to receive and compute the temperature signal from the temperature sensor and/or to activate the flow controller accordingly. This is intended to mean that preferably the controller can active the flow controller or parts thereof depending on the temperature detected or temperature signal representing a certain temperature.

The temperature controller and/or the flow controller and/or the controller or any two of these elements can be modular components that are adapted to be electrically and/or electronically and/or fluidly connected to each other with sockets. The sockets are preferably standardized so that the components fit to each other and/or can even be interchanged in the order.

The temperature controller can be suitable to provide infusion fluid at a temperature of between -1 °C and 40°C.

The flow controller can comprises a pump. Moreover an electronic controller con be adapted to stop the pump for a preset or given time and preferably to restart the pump after having received and computed the temperature signal from the temperature sensor after the preset or given time and a preset or given threshold temperature is reached or exceeded.

The device and/or method can also comprises a reservoir for taking up an infusion bag or other infusion container for bringing the infusion fluid to the temperature as mentioned.

Further the device comprises at least one temperature sensor suitable to deliver at least one temperature signal. This can be any kind of sensor available in the market. At least one pump is arranged that is suitable to pump the infusion fluid in an average amount of between 100 ml/h and 10 l/h. The pump can be any kind of pump available in the market, such as a peristaltic pump, piston pumps etc. Further, at least a first output such as an outgoing duct is provided for delivering the infusion fluid downstream the reservoir.

At least one controller is arranged or implemented which is able and adapted to receive and compute the temperature signal from the temperature sensor. It also is adapted to activate the pump if or in case the temperature signal corresponds to at least a preset threshold temperature. This is meant to embrace any scenario where the temperature is at or over a threshold value.

The pump is then activated over a preset or given time so that the pump delivers infusion fluid in a preset or given amount to the first outgoing duct or any other kind of output. The pump can be optionally stopped after and/or for for a preset or given time. The control can be further adapted to receive and compute the temperature signal after this preset or given time and repeat steps mentioned before. This is particularly intended to deliver further infusion fluid in case a desired temperature has not been reached.

The device can also comprise an assembly controller for controlling at least one component of the device. The assembly controller can be a modular component.

The device can further or comprise a temperature controller for tempering the infusion fluid and/or changing its temperature. The temperature controller can be a modular component and can alternatively or additionally be integrated with the assembly controller.

The device can also comprise a flow controller for controlling the flow of the infusion fluid. The flow controller can be a modular component and can alternatively or additionally be integrated with the assembly controller and/or temperature controller.

The temperature controller can be provided with at least a cooling section for cooling the infusion fluid and/or a heating section for heating the infusion fluid. The can be different temperature controllers for different needs in standardized manner.

The cooling and heating sections can be arranged in parallel and/or arranged in series.

The temperature controller can further comprise a neutral section for not influencing the temperature of the infusion fluid. The neutral section is adapted to allow a second infusion fluid to pass the temperature controller. This can be useful in case a second infusion fluid is used which either is not intended to be modified in temperature or which is temperature sensitive.

The pump can adapted to deliver the cold infusion fluid continuously and/or intermittently and/or sequentially, the latter preferably on the basis of pulses and intermediate pauses with volumes during the pulses of between 1ml to 50ml.

The device can be further adapted to deliver infusion fluid with a continuous, intermittent and/or sequential flow rate of 40 to 125 ml/h and/or a volume of 960 ml to 3000 ml per day and/or a continuous, intermittent and/or sequential flow rate of more than 125 ml/h. Thus, a basically continuous or base rate of infusion fluid and/or a bolus dosage (more volume than a continuous or base rate over the same or a shorter period of time) shall be delivered.

The continuous or base rate and/or the bolus dosage can be delivered together over the first outgoing duct or separately over a second outgoing duct. The device can be adapted to deliver infusion fluid with a continuous, intermittent and/or sequential flow rate of 40 to 125 ml/h and/or a volume of 960 ml to 3000 ml per day to the first outgoing duct and a continuous, intermittent and/or sequential flow rate of more than 125 ml/h to the second outgoing duct.

The first outgoing duct and/or the second outgoing duct can be adapted to deliver infusion fluid to a central venous catheter (CVC) and/or to a peripheral venous catheter (PVC) together and/or separately. In the latter case, e.g, the first outgoing duct delivers infusion fluid to the CVC and the second outgoing duct delivers infusion fluid to the PVC, or vice-versa.

Additionally or alternatively, the flow controller can be adapted to receive infusion fluid and/or a second infusion fluid directly from one of more reservoirs and to deliver it to at least one or more output duct(s). This is particularly useful when optionally delivering a common infusion fluid with a base rate and/or a temperature controlled or cooled infusion fluid in a bolus dosage to a central venous catheter (CVC) and/or the peripheral venous catheter (PVC).

The device can also comprise a central venous catheter (CVC) and/or to a peripheral venous catheter (PVC).

The temperature sensor can be suitable for measuring the temperature of blood, brain and/or esophagus of a patient and to deliver the temperature signal.

The preset threshold temperature can be at least 36°C and at most 38°C, preferably it is at least 36.9°C and more preferably 37.5°C.

The device and the method can alternatively comprise a preset threshold temperature of at least around 32°C to stop delivery of infusion fluid and at most around 34°C to (re-)start delivery of infusion fluid at least for given or pre-set time. This would particularly assist in hypothermia treatment. Preferably the temperature is then kept for around or exactly 12 to 24 hours and to further preferably then increase the temperature by around or exactly 0.25°C/h to 0.5°C/h until a preset temperature, such as normal physiological body temperature, preferably of around 37°C, is reached.

The preset amount of cold infusion fluid can be at least 0.1 l and at most 4.0 l, preferably at most 2.0 l.

The preset time period can be at least 1 min and at most 6 h.

Furthermore a display can be arranged at any of the components, preferably to the controller for the information of a user and/or manipulation preferably of the controller by a user.

The controller can comprise a storage for storing the temperatures detected and/or the pump activities and/or infusion amounts delivered and a display can be provided for displaying this information. Any other information component, such as a printer or just an interface for a central display or any other output device, can be provided.

The controller or assembly controller can be adapted at least receive input signals from at least one external computer system or to communicate with such system, such as an electronic patient file system.

The method according to the present invention can be particularly suitable for using a device described before or below or claimed below. It comprises the steps to receive and compute the temperature signal from the temperature sensor, to activate the pump if the temperature signal corresponds to at least a preset threshold temperature over a preset or given time period so that the pump delivers infusion fluid in a preset or given amount, to thereafter stop the pump (29) for a preset time or given time period and/or to receive and compute the temperature signal after the preset or given time and repeat the steps mentioned before.

The infusion fluid can be delivered in an intitial minimum amount of 0.8 l, preferably 0.9 l, more preferably 1.0 l and/or a maximum amount of 3.0 l, preferably 2.5 l, more preferably 2.0 l, more preferably 1.5 and most preferably 1.0 l. After this initial bolus rate subsequently between 100 ml and 1.0 l infusion fluid with flow rates of between 40ml/h and 8000 ml/h can be delivered.

The infusion fluid can be delivered with a minimum flow rate of 2000 ml/h, more preferably 3000 ml/h, even more preferably 4000 ml/h and/or a maximum flow rate of 7000 ml/h, preferably 6000 ml/h, more preferably 5000 ml/h and even more preferably 4000 ml/h.

The infusion fluid can be delivered by the method, device and/or flow controller for a minimum time period of 1 min, preferably 2 min, more preferably 3 min, more preferably 5 min, more preferably 10 min, more preferably 15 min, more preferably 20 min, more preferably 25 min, more preferably 30 min and/or a maximum amount of 90 min, preferably 40 min, more preferably 35 min, more preferably 30 min, more preferably 20 min. more preferably 15 min, more preferably 10 min and more preferably 5 min.

An antipyretic pharmaceutical can be also delivered.

The method can also comprise the further step of delivering infusion fluid to a central venous catheter (CVC) and/or infusion fluid to a peripheral venous catheter (PVC). It is referred to the respective description above. A continuous, intermittent and/or sequential flow rate of 40 to 125 ml/h and/or a volume of 960 ml to 3000 ml per day and a continuous, intermittent and/or sequential flow rate of more than 125 ml/h, preferably at least 2000 ml/h and at most 8000 ml/h, can be delivered to the central venous catheter (CVC) and/or the peripheral venous catheter (PVC). The delivery over one outgoing duct or two or more separate outgoing ducts can realize this.

When infusion fluid is delivered by a base rate of 40 ml/h to 125 ml/h it can be warmed up to preferably around 37.2 °C until a threshold temperature is detected. It can then be cooled down when the threshold has been detected. Additionally or alternatively another infusion fluid and/or the same infusion fluid can be delivered which is additionally cooled and delivered with the bolus rate of more than 125 ml/h, preferably even more than 2000 ml/h etc. as described before and below.

All aspects of the present invention are adjusted to operate or be operated without a patient. According to one aspect of the present invention the infusion fluid can be collected by a container for constant or test purposes or can be infused into a patient.

The preferred advantage of the present invention is to generate faster and further preferably more precisely adjusted or positively controlled temperatures of the infusion fluid. Thus, more individualized and a better adjusted flow of infusion fluids can be realized or a patient can be treated better according to the needs detected in real time or close to real time.

### Preferred embodiments

The present invention will become more fully understood from the description before and particularly below and the accompanying drawings that are given by way of illustration only and show and/or exemplify preferred aspects thereof, and wherein
**Fig. 1** is a principal sketch of a first embodiment of the present invention with a plurality of optional components;
**Fig. 2** shows an example of an embodiment of an assembly controller according to the invention;
**Fig. 3** shows an example of an embodiment of an a temperature controller according to the invention;
**Fig. 4** shows an example of an embodiment of a flow controller according to the invention; and
**Fig. 5** shows an example of an embodiment of an assembly controller, a temperature controller and a flow controller in a rack;
**Fig. 6** shows a further example of an arrangement of components or elements according to the present invention; and
**Fig. 7** shows a comparison of several examples of a temperature at a sensor and a given flow volume at the exit of a duct.

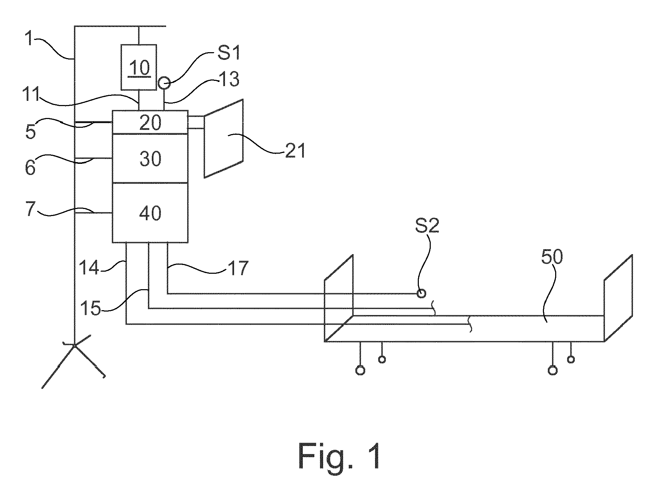

**Fig. 1** exemplifies one aspect of the present invention. A source or reservoir **10** of infusion fluid can be hung up or placed in any manner in order to deliver infusion fluid to a patient (not shown) or a container (not shown) of any kind of other element for testing or other purposes. In the embodiment shown the reservoir **10** is hung up an infusion holder **1** sometimes also sloppily called "Christmas tree". However it can be hung or supported in many different ways and even doesn't necessarily need to hang at any or any given height above the ground.

The reservoir **10** can be covered with or function as a thermal insulation or thermal treatment device in case the infusion fluid contained is preferred to be kept at temperatures different from room temperature.

Another or a plurality of reservoir(s) **10** can also be provided to provide either different infusion fluids and/or the same infusion fluids for different purposes and/or with different temperatures. One particular and non-exhaustive example is the provision of infusion fluids to different parts of a patient. This is explained in more detail before and/or below.

The infusion fluid is delivered by a reservoir duct **11** or pipe **11.** In case of more than one reservoir a respective or - in case of a combined delivery of fluids - a smaller number of ducts (not shown) may also be provided. For reasons of simplicity just one reservoir duct **11** is shown in **Fig. 1****.**

Downstream and/or below the reservoir **10** one or more device(s) **20** to **40** can be arranged for the further control of the infusion(s) and/or temperature(s) and/or flow rate(s) of the infusion fluid(s). The separation or modularity of components **20** to **40** shown in **Fig. 1** is preferred and can also be combined in one or more than one device or assembly.

In the embodiment shown an assembly controller **20** is shown which can (but most not) control one or all the further components **30, 40** described. The assembly controller **20** can be supported and hold in place with a assembly controller holder **5,** in the embodiment shown at the infusion holder **1.** However, it can also be arranged in a different manner, such as in a rack (not shown). Moreover it can comprise one or more displays **21** for the information and/or manipulation of a user.

A first sensor **S1** or a plurality of first sensors **S1** can be arranged in, at and/or adjacent the reservoir **10** in order to sense, determine and/or measure the parameters of the infusion fluid contained in the reservoir **10,** such as its temperature, composition, volume, level etc. The sensor **S1** can be connected to the assembly controller **20** by a wire **13** and/or wireless.

Connected or even attached to the assembly controller **20** is a temperature controller **30,** preferably downstream. Alternatively the assembly controller **20** can be arranged in parallel to the temperature controller and/or any further controller. The temperature controller **30** is essentially but not necessarily used to control and/or influences the temperature of the infusion fluid approaching from the reservoir(s) **10** through the duct(s) **11.** The temperature controller **30** can be supported and hold in place with a temperature controller holder **6,** in the embodiment shown at the infusion holder 1. However, it can also be arranged in a different manner, such as in a rack (not shown).

Downstream of the temperature controller **30** a flow controller **40** can be arranged. Alternatively or additionally the or any further flow controller 40 can be arranged upstream the temperature controller **30.** The flow controller is primarily but not necessarily used to control the flow rate and/or flow pressure to a container or a patient (both not shown). The flow controller **40** can be supported and hold in place with a flow controller holder 7, in the embodiment shown at the infusion holder **1.** However, it can also be arranged in a different manner, such as in a rack (not shown).

In the embodiment shown a first outgoing duct **14** is shown leading to a container or patient delivering infusion fluid with the temperature and/or flow rate being controlled. Additionally a second and optionally further outgoing duct(s) **15** can deliver infusion fluid with a different temperature and/or different flow rate.

Optionally a second sensor **S2** or a set of second sensors **S2** are provided to sense, determine and/or measure any parameters of interest for the control of the infusion fluid, its temperature and/or flow rate. It can measure the temperature of a container or patient and/or further conditions, such as other parameters. The second sensor(s) **S2** can be connected to the assembly controller **20** by a wire **17** and/or wireless.

For the better illustration a bed **50** for a patient (not shown) is also depicted in **Fig. 1****.**

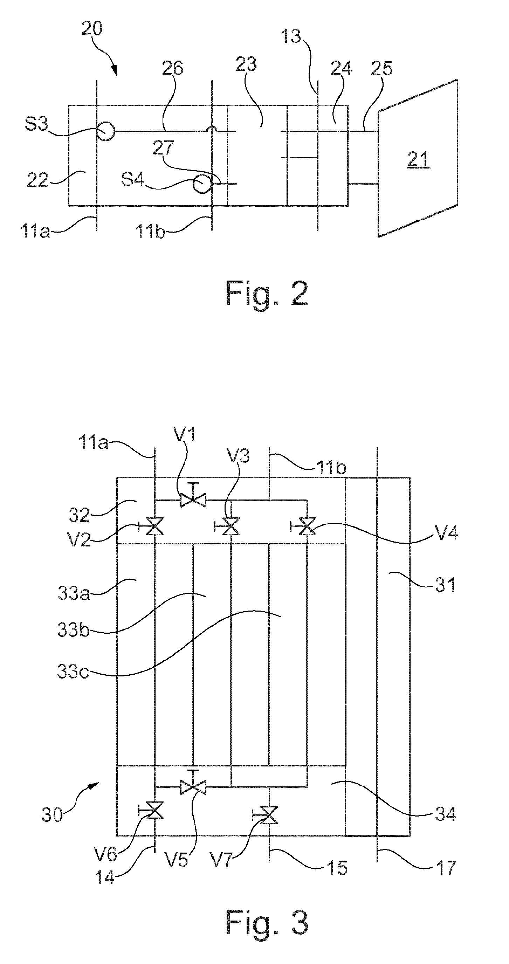

**Fig. 2** shows an example of an assembly controller **20.** It can have a flow section **22** through which the infusion fluid(s) are led by one duct **11** and/or more ducts **11a,11b.** An electronic controller **23** can be arranged as well one or more interfaces and/or controller connector(s) **24.** A connection **25** can connect the display **21** by hard wire and/or wireless with the electronic controller **23.** The electronic controller **23** can be of any known kind with a CPU, one or more storage etc. (not shown).

A third sensor **S3** can sense, determine and/or measure conditions, such as presence of the duct **11a** and/or temperature, flow rate and/or pressure etc. in the duct **11** or one of the ducts **11a.** The third sensor **S3** can be connected to the electronic controller **23** by a wire **26** and/or wireless. A fourth sensor **S4** can sense, determine and/or measure conditions, such as presence of the duct **11** and/or temperature, flow rate and/or pressure etc. in one of the ducts **11 b.** Further sensors can be present but are not shown. The fourth sensor **S4** can be connected to the electronic controller **23** by a hard wire **27** and/or wireless.

**Fig. 3** shows an example of the temperature controller **30.** It can comprise a temperature control connector **31** connecting the temperature controller **30** with the assembly controller **20** and/or any other components. As an example a connector line **17** is drawn in this temperature control connector **31** which can connect the temperature controller **30** with the assembly controller **20** preferably arranged above and/or one or more further controller(s) arranged below and/or above. The connector line **17** shown is intended to just exemplify one or more lines being connected to one or more of sections **32, 33, 34,** and/or one or more of valves **V1** to **V7** (described later in more detail). The connector line **17** can be of any kind, such as a bus connection line. Other units, components and/or controller may also be arranged in parallel and/or in series. The temperature control connector **31** can be hard wired and/or wireless and can have plugs and/or connectors at its end to ensure a connection when the temperature controller is arranged in place.

An incoming duct control section **32** can be arranged which can be controlled preferably by the assembly controller **20** in order to control the distribution of the infusion fluid(s) downstream in the temperature controller **30.** For this reason one or more or a plurality of valves **V1** to **V4** are shown. In one preferred embodiment a first duct **11a** delivers a first or a part of an infusion fluid and a second duct **11 b** delivers a second or a part of the first infusion fluid. With optional control valve **V1** they can be merged or kept separated or mixing amounts can be controlled. E.g., the separation of the two incoming first and second ducts **11a,b** can be realized in case a second reservoir (not shown) is positioned or hung up with an infusion fluid which is supposed to be delivered with room temperature, either as the infusion fluid should not have room temperature and/or as the way of delivery to a patient requires room temperature. In case of one reservoir of infusion fluid not being heated and/or cooled the present device or assembly is able to also control the delivery of further infusion fluids in a centralized manner. Further optional valves **V2, V3** and/or **V4** can (but most not) control the delivery of the infusion fluids to the further sections.

A tempering section (heating and/or cooling section) can comprise a neutral section **33a,** a heating section **33b** and/or a cooling section **33c.** Instead or additionally two or more cooling and/or heating stages with different cooling or heating cabilities can be arranged. The user and/or the assembly controller (not shown in Fig. 3) can control the valves **V1** to **V4** according to the needs of the infusion fluid(s) to be tempered. In the embodiment shown each valve **V2, V3, V4** can either allow or block or control the amounts to be further processed.

In an optional outgoing duct control section **34** the further delivery of the infusion fluids can (but must not) be controlled. The fluids leaving the neutral section **33a,** the heating section **33b** and/or the cooling section **33c** can be further mixed, merged or left unmixed by valves **V5** to **V7** in order to allow a further delivery and/or amounts according to the needs. In the embodiment shown a first outgoing duct **14** can be provided and an optional second outgoing duct **15** and/or any further outgoing ducts (not shown) can be provided. Just one of them or more outgoing ducts can also be arranged. With optional control valve **V5** they can be merged or kept separated or amounts can be mixed. The same or similar applies for the further valves **V6** and/or **V7** which can directly allow, permit or control amounts to go to the first outgoing duct **14** and/or second outgoing duct **15** or any further ducts (not shown).

In the temperature controller **30** common or separate pumps can be provided (not shown in the present embodiment). This holds particularly true in case there is a severe pressure drop either before, in or immediately after the temperature controller **30.** Such pump can be provided at either duct or line in front of, through and/or behind one or more of the sections **32, 33, 34** described.

The temperature controller **30** can also provide feed-back or closed loop controls or controlling sections which can particularly cooperate with the assembly controller **20** and/or sensors which are shown or not shown.

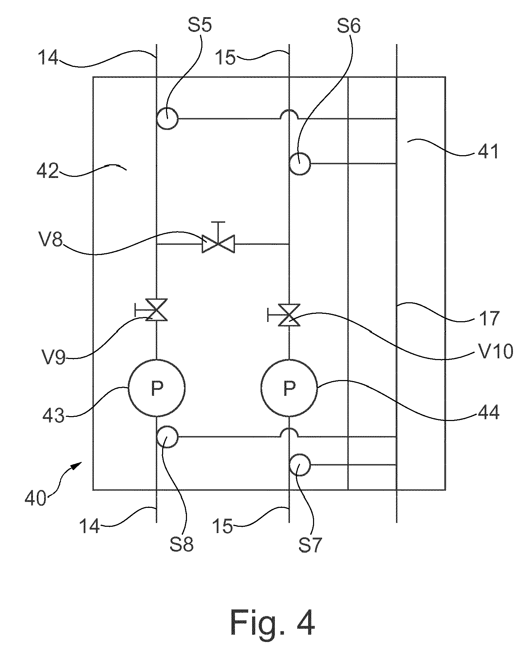

The embodiment shown in **Fig. 4** exemplifies a flow controller **40** which can be particularly adapted to control the flow of infusion fluid(s) (amount, pressure etc.) Similar to the description above a flow control connector can be provided to connect the flow controller to the assembly controller **20** or any other element or unit. For reasons of simplicity a flow control connector **41** with connector line 17 is drawn in this temperature control connector 31 which can connect the flow controller **40** with the assembly controller **20** preferably arranged above and/or one or more further controller(s) arranged below and/or above. The connector line **17** is intended to just exemplify one or more lines being connected to one or more of all components contained in the flow controller and described in further detail below. The connector line can be of any kind, such as a bus connection line. The flow control connector 41 can be hard wired and/or wireless and can have plugs and/or connectors at its end to ensure a connection when the temperature controller is arranged in place.

Sensor **S5** and/or sensor **S6** and/or further sensors can be arranged and adapted to sense, determine and/or measure any parameters of interest of the infusion fluid(s) and/or the first outgoing duct **14** and/or second outgoing duct **15** and/or further ducts. Such parameters can be the temperature(s), flow rate(s), pressure(s) etc. of the infusion fluid(s) contained in the ducts **14, 15.** The same applies to sensor(s) **S8** and/or sensor(s) **S7** and/or further sensors (not shown). However, these may sense, determine and/or measure the parameter before the fluids and/or ducts **14, 15** are leaving the flow controller and/or whole assembly of devices according to the present invention.

In the embodiment shown a first pump **43** and/or a second pump **44** can be arranged for each outgoing duct **14, 15.** Just one pump for each or just for one duct can also be provided. In case of a peristaltic pump one actor can also activate the flow in each duct 14, 15 on two sides of the turning actuator. A valve V8 can allow to separate and/or merge and/or defining amounts to be mixed between the two ducts **14, 15.** A valve **V9** and/or a valve **V10** can further control the flow or amounts of flow either upstream and/or downstream the pumps **43, 44.**

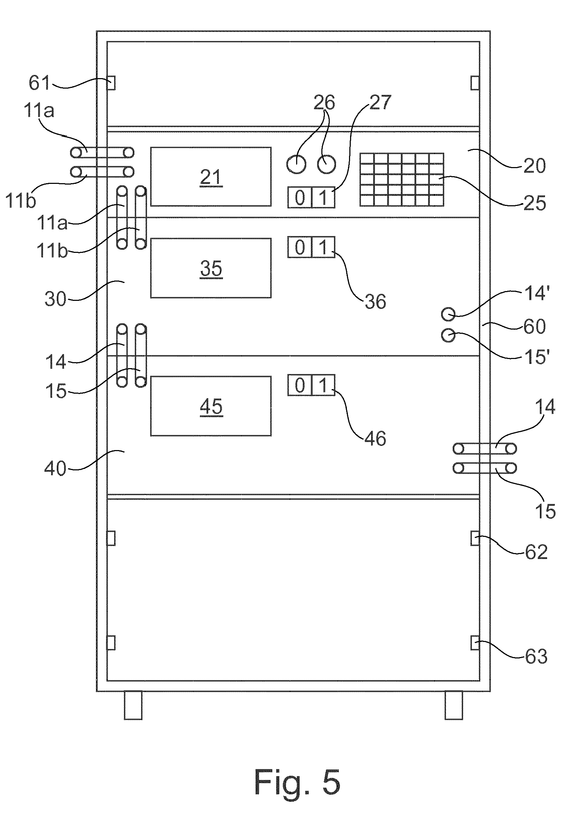

**Fig. 5** shows an example of an assembly of components **20, 30, 40** arranged in a rack **60.** The rack **60** can be oriented so that the components at issue **20, 30, 40** are arranged in a vertical orientation but can also be oriented to arrange them in a horizontal or inclined manner or any combination thereof. In such a rack **60** guide rails **61, 62, 63** can be arranged in order to allow any quick and reliable fixation of components or other elements therein. The guide rails **61, 62, 63** can have any other structure, or cannot be present. For the components **20, 30, 40** they are not shown.

The assembly controller **20** can be inserted into the rack **60** in the top-most position of all components **20, 30, 40** but can also be arranged in the middle or below the other components **30, 40.**

A first reservoir duct **11a** and a second reservoir duct **11 b** can be guided into the assembly controller **20,** e.g., in a plug-in configuration from any side. As an example the plug-in configuration is shown from the front so that a user can take these ducts coming from any reservoir (not shown in Fig.5) and plug them into any of the components **20, 30, 40,** preferably into the assembly controller **20.** In case a misconnection is to be avoided the ducts **11a, 11b** can be provided with plugs in customized form only fitting into sockets of the assembly controller **20.** In the embodiment shown the infusion fluid(s) coming from the reservoirs are guided into the assembly controller and finally leave in a further pair of ducts **11a,11b** and are introduced into the next element, in the example shown into the temperature controller **30.** Instead also the flow controller could be directly connected with the assembly controller **20.**

Any of the controllers **20, 30, 40** can also be connected with connectors being arranged in their neighboring surfaces, in the configuration shown the would be arranged in the bottom surface of assembly controller **20** and in the top surface of the element below, in the example shown the top surface of temperature controller **30.**

The ducts **11a, 11b** and/or the ducts **14, 15** can be of any type. E.g., they can be flexible and/or rigid and/or can be provided in the form of fixed connectors, particularly for the connection between the different components **20, 30, 40.** Also as indicated in the temperature controller **30** ports **14'** and **15'** can be provided for connecting any such ducts and/or rigid connectors in any of the components for any of the ducts **11a, 11b, 14, 15.**

The assembly controller **20** can comprise a monitor **21.** In case it cannot be used as a user input device or additionally a keyboard **25** can be also provided as a user interface. Moreover, other components can be provided such as turning knobs **26, 27** for changing parameters used often etc. An emergency know **27** can also be provided, like the ones **36, 46** in any other of the components **30, 40.**

The temperature controller **30** and/or the flow controller **40** can also comprise a monitor for monitoring and/or for user interface purposes.

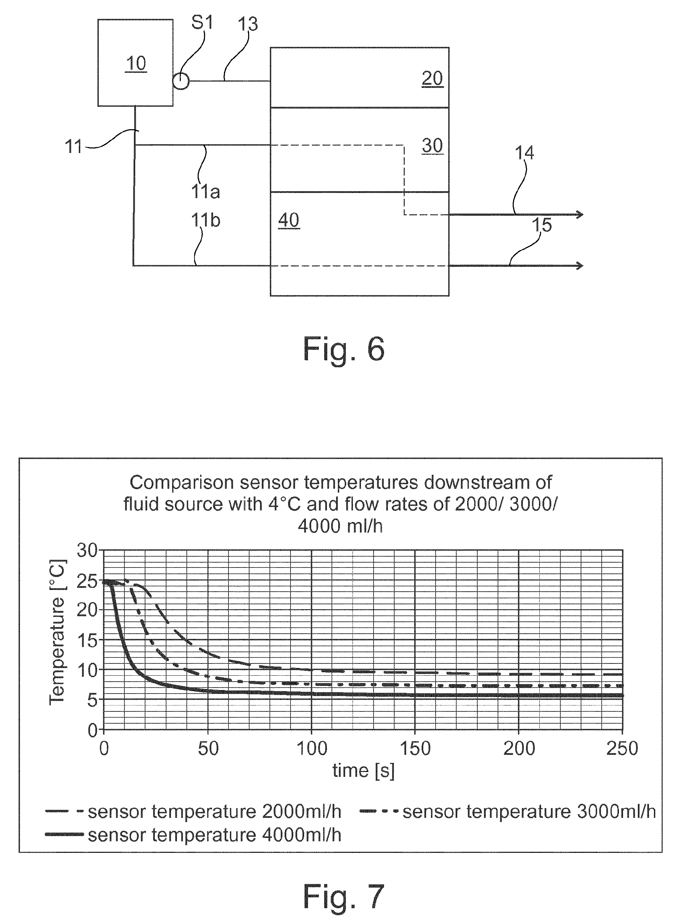

**Fig.** 6 shows another example for an arrangement of the components and connection to other elements in accordance with the present invention. Similar to the afore-described embodiments the components **20** to **40** can be arranged in a stack or in any other configuration. A sensor connection **13** can connect a sensor **S1** with the assembly controller **20.** Such a sensor is optional.

The reservoir duct **11** can split up to the first reservoir duct **11 a** and the second reservoir duct **11b.** As mentioned before, further branches can be realized. In the embodiments shown the first and second reservoir ducts are fed by the same reservoir **10.** Optionally or additionally one or more reservoirs can be provided feeding either one of the first or second reservoir duct or any other duct either with any one of the components **20** to **40** or can circumvent the components **20** to **40.** In the embodiment shown the first reservoir duct **11 a** enters the temperature controller **30** in order to allow its temperature to be controlled and/or modified. The respective infusion fluid can then be fed into the flow controller **40** and can leave this in a first outgoing duct **14.**

The second reservoir duct **11 b** can circumvent the temperature controller **30** and can directly go into the flow controller as it may not be necessary or even detrimental to modify its temperature. The respective infusion fluid may then leave in a second outgoing duct **14.**

The afore-described embodiment may be adapted to an infusion by means of a central venous catheter (CVC) and a separated infusion by a peripheral venous catheter (PVC). Either one may be fed by infusion fluid being temperature controlled or not temperature controlled. This can be particularly useful when delivering a bolus dosage with a rather high flow rate in temperature controlled form and a base rate with a lower flow rate in non-temperature controlled form. The bolus dosage can then go to either catheter, such as the central venous catheter in case a quickly effective cooling is desired. Examples of such dosages according to the present invention are mentioned before and below.

**Fig. 7** is intended to visualize the temperature losses from the reservoir of infusion fluids at different flow rates (2000/ 3000/ 4000 ml/h) over time. This shows the advantage of a feed-back or closed loop control and a respective compensation from the reservoir downstream any assemblies or devices. It is particularly apparent that the lower the flow rate is the higher the relative temperature loss is. This shows that higher flow rates allow a better control of the temperature of infusion fluids delivered to a container or a patient. In the arrangement of the embodiments tested the infusion fluid is warmed during flow in the ducts and the ambient atmosphere etc. After a certain amount of time, such as 50 s, the infusion fluid delivered is between 12.5°C and slightly above 6°C, for flow rates of 2,000 ml/h and 4,000 ml/h, respectively, while with further time the temperature of the infusion fluid further drops to between 9°C and slightly below 6°C, for flow rates of 2,000 ml/h and 4,000 ml/h, respectively.

Thus, it has been found that the present invention and aspects thereof enable a faster and further preferably more precisely adjusted or positively controlled temperatures of the infusion fluid. Thus, more individualized and a better adjusted flow of infusion fluids can be realized or a patient can be treated more according to the needs detected in real time or close to real time.
As used herein, including in the claims, singular forms of terms are to be construed as also including the plural form and vice versa, unless the context indicates otherwise. Thus, it should be noted that as used herein, the singular forms "a," "an," and "the" include plural references unless the context clearly dictates otherwise.

Throughout the description and claims, the terms "comprise", "including", "having", and "contain" and their variations should be understood as meaning "including but not limited to", and are not intended to exclude other components.

The present invention also covers the exact terms, features, values and ranges etc. in case these terms, features, values and ranges etc. are used in conjunction with terms such as about, around, generally, substantially, essentially, at least etc. (i.e., "about 3" shall also cover exactly 3 or "substantially constant" shall also cover exactly constant).

The term "at least one" should be understood as meaning "one or more", and therefore includes both embodiments that include one or multiple components. Furthermore, dependent claims that refer to independent claims that describe features with "at least one" have the same meaning, both when the feature is referred to as "the" and "the at least one".

It will be appreciated that variations to the foregoing embodiments of the invention can be made while still falling within the scope of the invention. Alternative features serving the same, equivalent or similar purpose can replace features disclosed in the specification, unless stated otherwise. Thus, unless stated otherwise, each feature disclosed represents one example of a generic series of equivalent or similar features.

Use of exemplary language, such as "for instance", "such as", "for example" and the like, is merely intended to better illustrate the invention and does not indicate a limitation on the scope of the invention unless so claimed. Any steps described in the specification may be performed in any order or simultaneously, unless the context clearly indicates otherwise.

All of the features and/or steps disclosed in the specification can be combined in any combination, except for combinations where at least some of the features and/or steps are mutually exclusive. In particular, preferred features of the invention are applicable to all aspects of the invention and may be used in any combination.

## Claims

1. Device, particularly suitable in a temperature adjustment infusion system and more particularly suitable for normothermia and/or hypothermia, comprising:
a. at least one connection (11) to a reservoir (10) suitable to provide infusion fluid;
b. at least one temperature controller (30) adapted to cool and/or heat the temperature of the infusion fluid so that the infusion fluid is delivered with a pre-set temperature at a temperature of between -1°C and 42°C;
c. at least one flow controller (40) adapted to control the flow downstream the temperature controller (30) in an average amount of between 100 ml and 8000 ml with an average flow rate of preferably 40 ml/h to 8000 ml/h; and
d. at least one output (14) adapted to deliver the infusion fluid downstream the reservoir.

2. Device according to claim 1 further comprising at least one temperature sensor (S2) suitable to deliver at least one temperature signal and at least one assembly controller (20) adapted to receive and compute the temperature signal from the temperature sensor (S2) and/or to activate the flow controller accordingly.

3. Device according to any one of the preceding claims wherein the temperature controller (30) and/or the flow controller (40) and/or the controller (20) or any two of these elements are modular components that are adapted to be electrically and/or electronically and/or fluidly connected to each other with sockets.

4. Device according to any one of claims 2 to 4 wherein the flow controller (40) comprises a pump (29) and wherein the controller (20) is adapted to stop the pump (29) for a preset or given time and preferably to restart the pump (29) after having received and computed the temperature signal from the temperature sensor (12) after the preset or given time and a preset or given 4temperature is reached or exceeded.

5. Device according to any one of the preceding claims wherein the temperature controller (30) comprises at least one cooling section (33b) adapted to cool infusion fluid and at least one heating section (33c) adapted to heat infusion fluid, the cooling section and the heating section (33b, 33c) being arranged in parallel and/or in series.

6. Device according to any one of the preceding claims wherein the temperature controller (30) further comprises a neutral section (33a) for not influencing the temperature of the infusion fluid.

7. Device according to any one of the preceding claims wherein the neutral section (33a) is adapted to allow a second infusion fluid to pass the temperature controller (30).

8. Device according to any one of the preceding claims wherein the flow controller (40) is adapted to deliver the infusion fluid(s) continuously and/or intermittently and/or sequentially, preferably on the basis of pulses and intermediate pauses with volumes during the pulses of between 1 ml to 50ml.

9. Device according to any one of the preceding claims wherein the device is adapted to deliver infusion fluid with a continuous, intermittent and/or sequential flow rate of 40 to 125 ml/h and/or a volume of 960 ml to 3000 ml per day and/or a continuous, intermittent and/or sequential flow rate of more than 125 ml/h.

10. Device according to any of the preceding claims further comprising a second outgoing duct (15) wherein the device is adapted to deliver infusion fluid with a continuous, intermittent and/or sequential flow rate of 40 to 125 ml/h and/or a volume of 960 ml to 3000 ml per day to the first outgoing duct (14) and a continuous, intermittent and/or sequential flow rate of more than 125 ml/h to the second outgoing duct (15).

11. Device according to claim 10 wherein the first outgoing duct (14) and/or the second outgoing duct (15) is/are adapted to deliver infusion fluid to a central venous catheter (CVC) and/or to a peripheral venous catheter (PVC), respectively.

12. Device according to claim 10 or 11 further comprising a central venous catheter (CVC) and/or to a peripheral venous catheter (PVC).

13. Device according to any one of claims 2 to 12 wherein the temperature sensor (S2) is suitable for measuring the temperature of blood, brain and/or esophagus of a patient and to deliver the temperature signal.

14. Device according to any one of claims 4 to 13 wherein the preset threshold temperature is at least 36.9°C and more preferably 37.5°C.

15. Device according to any one of the preceding claims wherein the preset threshold temperature is at least around 32°C to stop delivery of infusion fluid and at most around 34°C to (re-)start delivery of infusion fluid at least for given or pre-set time, and the device being preferably adapted to keep this temperature for around 12 to 24 hours and to further preferably then increase the temperature by around 0.25°C/h to 0.5°C/h until a preset temperature, such as normal physiological body temperature, is reached.

16. Device according to any one of the preceding claims further comprising a display (21) for the information of a user and/or manipulation of the controller (20) by a user.

17. Device according to any one of the preceding claims wherein the controller (20) comprises a storage for storing the temperatures detected and/or the pump activities and/or infusion amounts delivered and a display (21) for displaying this information.

18. Device according to any one of the preceding claims wherein the flow controller (40) is additionally or alternatively adapted to receive one or more infusion fluid(s) directly from reservoirs and to deliver it to one ore more output duct(s) (15).

19. The device according to any of the preceding claims, wherein the controller (20) or assembly controller (20) is configured to receive input signals from at least one external computer system and/or to communicate with such system, such as an electronic patient file system.

20. Device according to any one of the preceding claims wherein the flow controller (40) is adapted to feed a first outgoing duct (14) with a first base flow rate and a second outgoing duct (15) with a bolus flow rate being higher than the first base flow rate.

21. Device according to any one of the preceding claims wherein the device is adapted to deliver infusion fluid with a continuous, intermittent and/or sequential base flow rate of 40 to 125 ml/h and/or a volume of 960 ml to 3000 ml per day and/or a continuous, intermittent and/or sequential bolus flow rate of more than 125 ml/h.

22. Device according to any of the preceding claims further comprising a first outgoing duct (14) and second outgoing duct (15) wherein the device is adapted to deliver infusion fluid with a continuous, intermittent and/or sequential base flow rate of 40 to 125 ml/h and/or a volume of 960 ml to 3000 ml per day to the first outgoing duct (14) and a continuous, intermittent and/or sequential bolus flow rate of more than 125 ml/h to the second outgoing duct (15).

23. Device according to any of the preceding claims further comprising a first outgoing duct (14) and a second outgoing duct (15) wherein the device is adapted to deliver infusion fluid with a continuous, intermittent and/or sequential base flow rate of 40 to 125 ml/h and/or a volume of 960 ml to 3000 ml per day to the first outgoing duct (14) and a continuous, intermittent and/or sequential bolus flow rate of more than 2000 ml/h to the second outgoing duct (15).

24. Device according to any of claims 20 to 23 wherein the device is adapted to deliver the bolus flow rate amounts to at least 2000 ml/h to 4000 ml/h.

25. Device according to any one of the preceding claims wherein the flow controller (40) is adapted to deliver the infusion fluid in a minimum time period of 1 min, preferably 2 min, more preferably 3 min, more preferably 5 min, more preferably 10 min, more preferably 15 min, more preferably 20 min, more preferably 25 min, more preferably 30 min and/or a maximum amount of 90 min, preferably 40 min, more preferably 35 min, more preferably 30 min, more preferably 20 min. more preferably 15 min, more preferably 10 min and more preferably 5 min.

26. Device according to any one of the preceding claims wherein at least a first branch (11 a) or first reservoir duct (11a) of the connection (11) to the reservoir (10) is adapted to feed infusion fluid through a neutral section (33a) in the temperature controller (30) and/or directly into the flow controller (40) so that the infusion fluid can be fed into the first outgoing duct (14) and/or wherein at least a second branch (11a) of the connection (11) to the reservoir (10) or second reservoir duct (11b) is adapted to feed infusion fluid through the temperature controller (30) which is adapted to deliver downstream temperature controlled infusion fluid.

27. Device according to any one of claims 20 to 26 wherein the device is adapted so that at least the first of the outgoing duct (14) is fed with infusion fluid with the base flow rate and the second outgoing duct (15) is fed with infusion fluid with the bolus flow rate.

28. Method, particularly suitable in a temperature adjustment infusion system and more particularly suitable for normothermia and/or hypothermia and particularly suitable for using a device according to any one of the preceding claims, comprising the following steps:
a. obtaining infusion fluid by at least one connection from a reservoir (suitable to provide infusion fluid;
b. cooling the temperature of the infusion fluid sufficiently so that the infusion fluid is delivered with a pre-set temperature at a temperature of between -1°C and 40°C by at least one temperature controller;
c. controlling the flow downstream the temperature controller in an average amount of between 100 ml and 8000 ml with an average flow rate of preferably 40 ml/h to 8000 ml/h by at least one flow controller and
d. delivering the infusion fluid downstream the reservoir by at least one output.

29. Method according to any one of the preceding method claims wherein the infusion fluid is initially delivered in a minimum amount of 0.8 l, preferably 0.9 l, more preferably 1.0 l and/or a maximum amount of 3.0 l, preferably 2.5 l, more preferably 2.0 l, more preferably 1.5 and most preferably 1.0 l, and/or preferably and subsequently between 100 ml and 1.0 l with flow rates of between 40ml/h and 8000 ml/h.

30. Method according to any one of the preceding method claims wherein the infusion fluid is delivered with a minimum flow rate of 2000 ml/h, more preferably 3000 ml/h, even more preferably 4000 ml/h and/or a maximum flow rate of 7000 ml/h, preferably 6000 ml/h, more preferably 5000 ml/h and even more preferably 4000 ml/h.

31. Method according to any one of the preceding method claims wherein the infusion fluid is delivered for a minimum time period of 1 min, preferably 2 min, more preferably 3 min, more preferably 5 min, more preferably 10 min, more preferably 15 min, more preferably 20 min, more preferably 25 min, more preferably 30 min and/or a maximum amount of 90 min, preferably 40 min, more preferably 35 min, more preferably 30 min, more preferably 20 min. more preferably 15 min, more preferably 10 min and more preferably 5 min.

32. Method according to any one of the preceding method claims with the further step of delivering infusion fluid to a central venous catheter (CVC) and/or infusion fluid to a peripheral venous catheter (PVC).

33. Method according to any one of the preceding method claims with the further step of delivering infusion fluid with a continuous, intermittent and/or sequential flow rate of 40 to 125 ml/h and/or a volume of 960 ml to 3000 ml per day and a continuous, intermittent and/or sequential flow rate of more than 125 ml/h, preferably 2000 ml/h to 8000 ml/h, to the central venous catheter (CVC) and/or the peripheral venous catheter (PVC), respectively.

34. Method according to any of the preceding method claims wherein the infusion fluid delivered by a base rate of 40 ml/h to 125 ml/h is warmed up to around 37.2 °C until the threshold is detected, an then is cooled down when the threshold has been detected and optionally another infusion fluid and/or the same infusion fluid is additionally cooled and delivered with the bolus rate of more than 125 ml/h.

35. Method according to any one of the preceding method claims wherein the preset threshold temperature is at least around 32°C to stop delivery of infusion fluid and at most around 34°C to (re-)start delivery of infusion fluid at least for given or pre-set time, and method being preferably adapted to keep this temperature for around 12 to 24 hours and to further preferably then increase the temperature by around 0.25°C/h to 0.5°C/h until a preset temperature, such as normal physiological body temperature, is reached.

36. Method of treating a mammal comprising using a device and/or a method according to any one of the preceding claims.
